# EUROPEAN PATENT APPLICATION

(11) **EP 1 488 800 A1**
(43) Date of publication of application: **22.12.2004**
(21) Application number: 03715387.1
(22) Date of filing: 20.03.2003
(51) Int. Cl.: A61K 38/22, A61K 9/08, A61P 7/06, A61P 33/02, A61P 33/04, A61P 33/06

(54) **AGENT FOR THE TREATMENT OF PROTOZOAL DISEASES**

(30) Priority: 22.03.2002 JP 2002080482
(71) Applicant: CHUGAI SEIYAKU KABUSHIKI KAISHA, Tokyo, 115-8543 (JP)
(72) Inventor: SUZUKI, Hiroshi, Obihiro-shi, Hokkaido 080-0028 (JP)
(74) Representative: VOSSIUS & PARTNER
(86) International application number: PCT/JP2003/003426
(87) International publication number: WO 2003/080107

(57) **Abstract**

An EPO-containing pharmaceutical composition effective for the treatment of protozoal disease, a method for treatment of protozoal disease by administering EPO to a patient with protozoal disease, and use of EPO in producing an agent for treatment of protozoal disease are disclosed.

Several medicines have been used for treatment of protozoal diseases, but these medicines have relatively high side effects. Pentamidine, in particular, has been considered to be effective against protozoal disease, but may develop severe hypotension, hypoglycemia and arrhythmia. In patients with disorder of the kidney or liver, pentamidine may aggravate impaired renal or hepatic function, and tends to show side effects. Pentamidine may also exacerbate the symptoms of patients with hypotension or hypertension, and patients with hypoglycemia or hyperglycemia. The present invention provides an agent for treatment of protozoal disease, the agent free from or minimal in such side effects.

## Description

### [TECHNICAL FIELD]

This invention relates to an agent for treatment of protozoal disease, which contains erythropoietin (EPO) as the active ingredient.

### [BACKGROUND ART]

Protozoal disease is a generic name for diseases which are caused by infections with various protozoa spread to humans or other animals. Known as typical protozoal diseases are diseases due to infections, such as trypanosomiasis, amebiasis, malaria, toxoplasmosis, pneumocystosis, cryptosporidiosis, giardiasis, and leishmaniasis.

Of these diseases, trypanosomiasis is also called sleeping sickness, and occurs in two known forms, African trypanosomiasis distributed mainly in Middle and East Africa, and American trypanosomiasis distributed in Central and South America and called Chagas' disease. The former disease is transmitted by tsetse flies, and the latter disease is transmitted by reduviid bugs. Either trypanosomiasis presents with symptoms, such as fever, headache, or lymph node enlargement, and results in death.

Currently, main therapies of trypanosomiasis include the administration of pentamidine, etc. However, these drugs may cause side effects, such as severe hypotension, hypoglycemia and arrhythmia, and their cautious administration is recommended.

In trypanosomiasis and malaria infections, anemia is observed as the main clinical and laboratory finding, but little is known about the pathogenesis of their associated anemia. In 1995, Buza et al. reported that calves infected by Trypanosoma congolense (hereinafter may be referred to as T. congolense) exhibited severe anemia, although their EPO concentrations in circulation increased. A similar observation in murine infection with virulent malaria (Plasmodium berghei) had been published three decades ago (Rencricca et al., Infection and Immunity 10, 831-833 (1974)). According to the aforementioned report of Buza et al., a possibility for the presence of inhibitory factors acting on the erythropoietic system has been strongly suggested. Hence, no attempts have been made to administer EPO for the treatment of anemia associated with protozoan infection including Trypanosoma. However, no conclusion has been reached as to whether the administration of exogenous EPO at a high dose resolves anemia due to protozoan infection. Reports issue to date have clarified that endogenous EPO does not resolve this type of anemia.

Several medicines have been used for the treatment of protozoal diseases, but these medicines have relatively high side effects. Pentamidine, in particular, has been considered to be effective against protozoal disease, but may develop severe hypotension, hypoglycemia and arrhythmia. In patients with disorder of the kidney or liver, pentamidine may aggravate impaired renal or hepatic function, and tends to show side effects. Pentamidine may also exacerbate the symptoms of patients with hypotension or hypertension, and patients with hypoglycemia or hyperglycemia. In the light of these circumstances, the development of a pharmaceutical effective against protozoal disease and minimal in side effects has been desired.

### [DISCLOSURE OF THE INVENTION]

We, the inventors of the present invention, have confirmed that anemia attributed to protozoal disease is ameliorated by the administration of EPO and, to our surprise, has found that the administration of EPO is effective for the treatment of protozoal disease itself. Based on this finding, we have developed an EPO-containing pharmaceutical composition effective for the treatment of protozoal disease.

### [BRIEF DESCRIPTION OF THE DRAWINGS]

FIG. 1 is a graph showing the effect of treatment with EPO (5,000 U/kg) on the survival rate of mice infected with Trypanosoma congolense.
FIG. 2 is a graph showing the effect of treatment with EPO (5,000 U/kg) on the hematocrit values of mice infected with Trypanosoma congolense.
FIG. 3 is a graph showing the effect of treatment with EPO (5,000 U/kg) on parasitemia of mice infected with Trypanosoma congolense.
FIG. 4 is a graph showing the effect of treatment with EPO (1,000 U/kg) on the survival rate of mice infected with Trypanosoma congolense.
FIG. 5 is a graph showing the effect of treatment with EPO (1,000 U/kg) on the hematocrit values of mice infected with Trypanosoma congolense.
FIG. 6 is a graph showing the effect of treatment with EPO (1,000 U/kg) on parasitemia of mice infected with Trypanosoma congolense.
FIG. 7 is a graph showing changes in the body weight of mice infected with Trypanosoma congolense.
FIG. 8 is a graph showing the effect of treatment with EPO (5,000 U/kg) and Pen (4 mg/kg) on the survival rate of mice infected with Trypanosoma congolense.
FIG. 9 is a graph showing the effect of treatment with EPO (5,000 U/kg) and Pen (4 mg/kg) on the hematocrit values of mice infected with Trypanosoma congolense.

### [MODES FOR CARRYING OUT THE INVENTION]

Protozoal disease in the present invention is a generic name for diseases which are caused by infections with various pathogenic parasitic protozoa spread to humans or other animals. Known as typical protozoal diseases are diseases due to infections, such as amebiasis, trypanosomiasis, malaria, toxoplasmosis, pneumocystosis, cryptosporidiosis, giardiasis, and leishmaniasis. The protozoal diseases, which are given particular attention as a target for the therapy of the present invention, are diseases accompanied by anemia, such as trypanosomiasis, malaria, babesiosis, and leishmaniasis. The present invention is effective, particularly, for the treatment of trypanosomiasis.

As will be concretely described in experimental examples later, when mice are infected with Trypanosoma, especially, Trypanosoma congolense highly pathogenic to cattle, at a rate of 1,000 cells per mouse, all animals died in a short period in a non-treatment group, whereas treatment with human recombinant EPO (r-hEPO) was confirmed to improve the lethality dramatically. Based on this finding, we developed an EPO-containing pharmaceutical for the treatment of protozoal diseases, especially, protozoal diseases involving anemia (e.g., trypanosomiasis and malaria), in humans or other animals. We confirmed its pharmacological effect, and accomplished the present invention.

A patient, who was infected with Trypanosoma, has a possibility of escaping death when infected, for the second time, with Trypanosoma of the same species as the species causing the first infection. Thus, EPO and Trypanosoma are simultaneously administered to avoid lethal infection with Trypanosoma. Such a combined therapy can be used as a vaccine.

As erythropoietin (EPO), the active ingredient used in the present invention, there can be named, for example, natural human EPO obtained by extraction from the urine of patients with human aplastic anemia (Japanese Patent Publication No. 1989-38,800); and EPO products manufactured by genetic recombination technologies which comprise collecting messenger RNA (mRNA) corresponding to the amino acid sequence of human EPO, constructing recombinant DNA with the use of the mRNA, and then causing suitable hosts (e.g., microorganisms such as Escherichia coli, yeasts, cell strains of plants, and cell strains of animals, such as COS cells, Chinese hamster ovarian cells (CHO), and mouse C-127 cells) to produce EPO from the recombinant DNA [for example, Japanese Patent Publication No. 1989-44317; Kenneth Jacobs et al., Nature, 313, 806-810 (1985)].

EPO usable in the present invention may be those of the above-mentioned origins, and their modifications. EPO modification products include, for example, those described in Japanese Patent Application Laid-Open No. 1991-151399. Among these EPO modification products are those in which Asn in the peptide chain of the original glycoprotein has been mutated to Gln, and the number of bindings of N-linked sugar chains bound has been changed. As other amino acid mutations, those described in Japanese Patent Application Laid-Open No. 1990-59599 and Japanese Patent Application Laid-Open No. 1991-72855 are named. That is, there may be any number of amino acid mutations, deletions, or additions, unless the property of EPO acting on the EPO receptor is lost.

In regard to preparations containing EPO of the present invention as the active ingredient, suspending agents, solution adjuvants, stabilizers, tonicity agents, preservatives, and adsorption preventing agents may be added, if desired, depending on the methods of administering them and their dosage forms. Examples of the suspending agents are methylcellulose, polysorbate 80, hydroxyethylcellulose, acacia, tragacanth powder, sodium carboxymethylcellulose, and polyoxyethylene sorbitan monolaurate. Examples of the solution adjuvants are polyoxyethylene hydrogenated castor oil, polysorbate 80, nicotinamide, polyoxyethylene sorbitan monolaurate, macrogol, and castor oil fatty acid ethyl ester. Examples of the stabilizers are human serum albumin, dextran 40, methylcellulose, gelatin, sodium sulfite, and sodium metasulfite. Examples of the tonicity agents are D-mannitol and sorbitol. Examples of the preservatives are methyl parahydroxybenzoate, ethyl parahydroxybenzoate, sorbic acid, phenol, cresol, and chlorocresol. Examples of the adsorption preventing agents are human serum albumin, lecithin, dextran, ethylene oxide-propylene oxide copolymer, hydroxypropylcellulose, methylcellulose, polyoxyethylene hydrogenated castor oil, and polyethylene glycol.

By using the EPO of the present invention as the active ingredient and adding a certain amino acid as a stabilizer, a stable EPO solution preparation free from human serum albumin or purified gelatin can be used. This stable EPO solution preparation is described in Japanese Patent Application Laid-Open No. 1998-182481. The descriptions in this publication are to be included in a part of the specification of the present application.

The amino acid added as the stabilizer in this EPO solution preparation includes free amino acids, and their salts such as sodium salts, potassium salts and hydrochlorides. To the solution preparation of the present invention, one of these amino acids can be added, or two or more of these amino acids can be added in combination. The preferred amino acids are leucine, tryptophan, serine, glutamic acid, arginine, histidine, and lysine, each in D-, L- and DL-forms, and their salts. More preferred are L-leucine, L-tryptophan, L-glutamic acid, L-arginine, L-histidine, and L-lysine, and their salts. Particularly preferred are L-arginine, L-histidine, and L-lysine, and their salts. The most preferred are L-histidine, and its salts.

Details of the method, etc. for producing the above-described stable EPO solution preparation are as described in the aforementioned publication.

The dose of these EPO products in prophylactic and therapeutic drugs targeted by the present invention can be determined, as appropriate, in consideration of the disease to be dealt with, its symptoms and so forth. The dose usually 0.1 to 500 µg, preferably 5 to 100 µg, per adult.

Hereinbelow, the present invention will be described in further detail by Experimental Examples and Examples, but the present invention is in no way limited by these Experimental Examples and Examples.

### [Experimental Example 1] Effect by treatment with r-hu-EPO

C57BL/6J female mice (CLEA, Tokyo, Japan) at 7 weeks of age were infected with 1,000 cells of T. congolense (International Livestock Research Institute, Kenya) by intraperitoneal injection (Day 0). The mice were treated daily with recombinant human erythropoietin (r-hu-EPO; Chugai Pharmaceuticals, Tokyo, Japan) at a dose of 5,000 U/kg (Experiment 1) or 1,000 U/kg (Experiment 2) for 8days from Day 0 to Day 7 of infection, and their survival rate, hematocrit values, and the number of protozoa per unit amount of the blood were monitored.

### (Results)

### Experiment 1

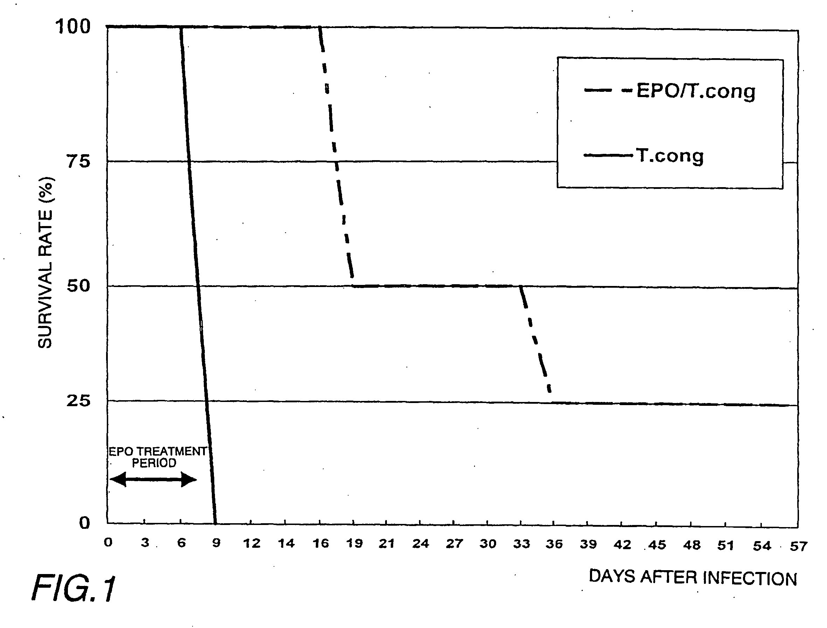

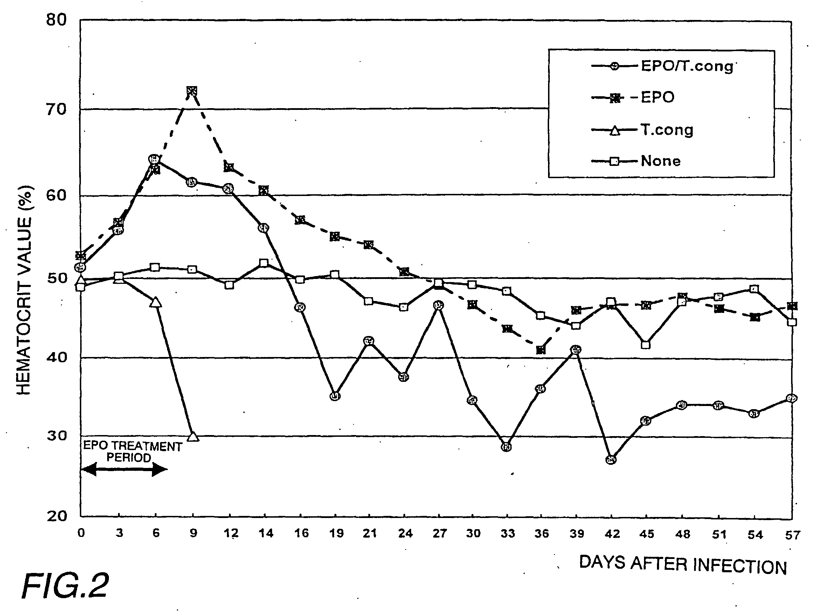

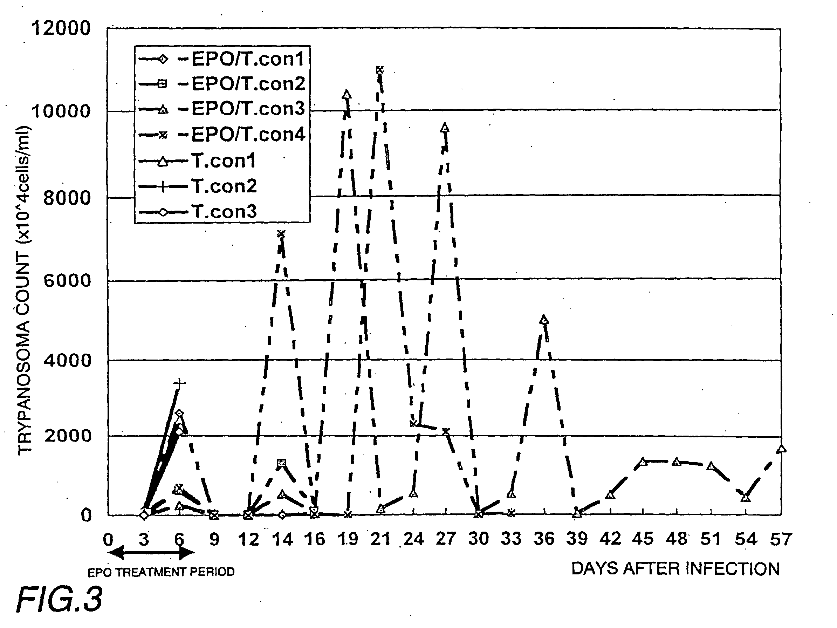

As shown in FIG. 1, all of the mice infected with T. congolense (1,000 cells) died by Day 9 of infection. However, all and 50% of the infected mice treated with 5,000 U/kg of r-hu-EPO still survived on Day 16 and Day 33 of the protozoal infection, respectively. Any clinical side effects were not detected in the mice receiving r-hu-EPO without protozoal infection, except for marked increases in the hematocrit values (FIG. 2). The hematocrit values of the infected mice treated with r-hu-EPO and the mice receiving r-hu-EPO without parasite infection similarly increased until Day 6. However, there was a tendency that the hematocrit values in the infected mice treated with r-hu-EPO were lower from Day 9 than those in the mice receiving r-hu-EPO. Furthermore, the hematocrit values of the infected mice treated with r-hu-EPO were much less than those of the untreated control mice from Day 16 onwards. Most of the mice infected with Trypanosoma did not show severe anemia during the course of infection. However, when one of the infected mice, which was debilitated and moribund, was sacrificed, its hematocrit value decreased to 30%. The increase in parasitemia (the presence of protozoa in the circulating blood) in surviving animals occurred as several distinct peaks rather than as a sustained increase. Nearly all of the infected mice receiving no EPO died at, or immediately after, the first peak of the parasitemia (2108-3413 × 10⁴ cells/ml blood; FIG. 3).

### Experiment 2

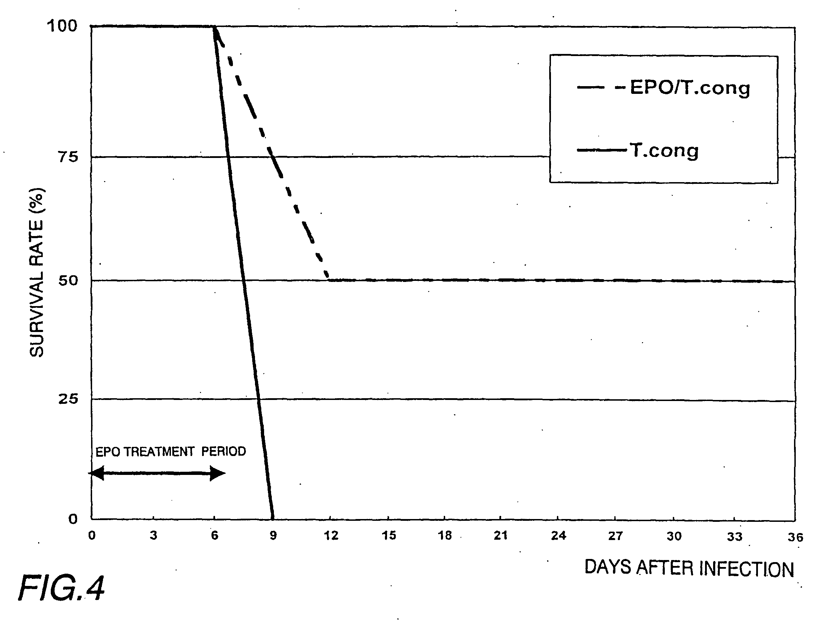

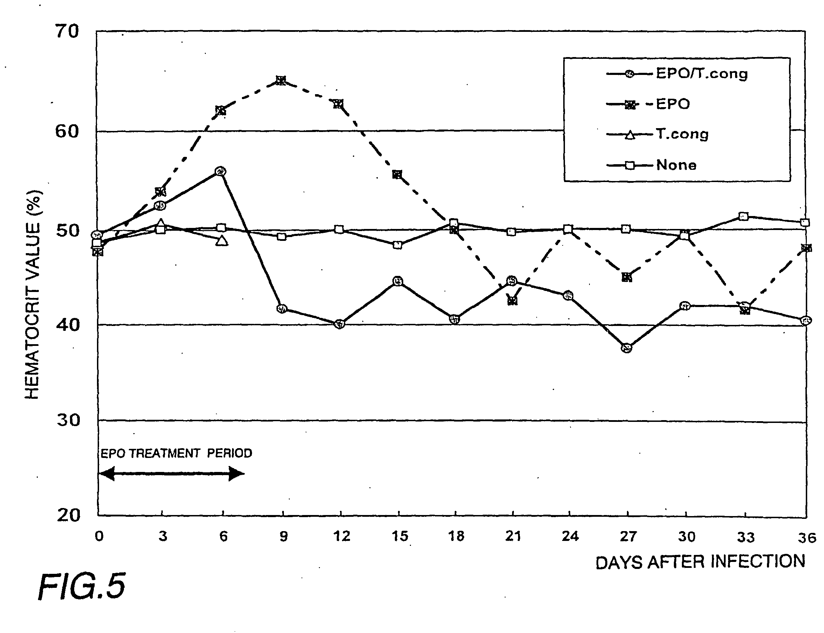

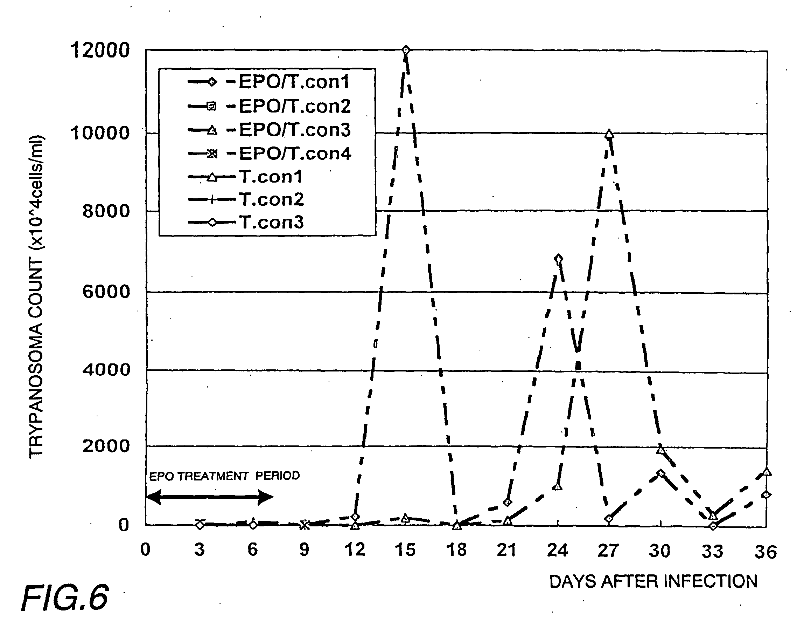

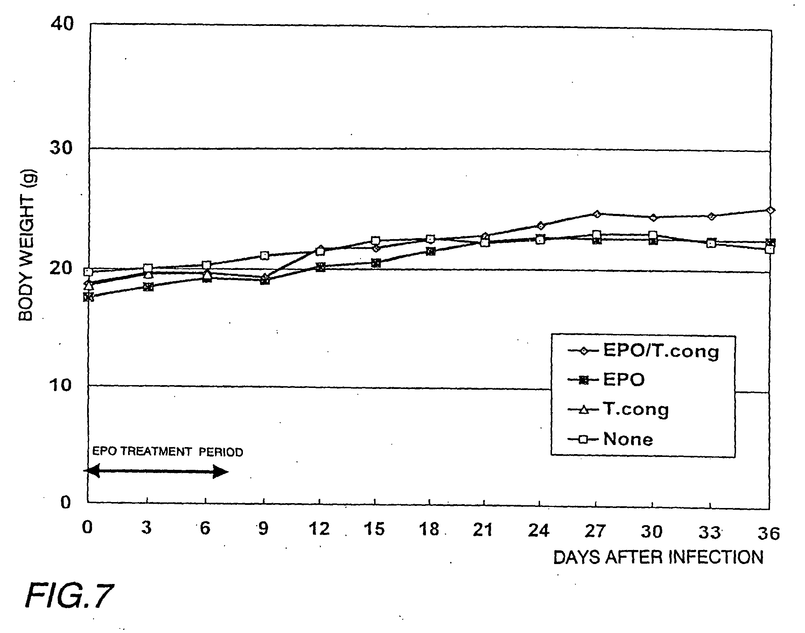

When mice were infected with T. congolense (1,000 cells), all of the mice died within 9 days as in the results of Experiment 1. On the other hand, the survival rate of the mice infected with T. congolense (1,000 cells) and treated with 1,000 U/kg of r-hu-EPO for 8 days was 75% on Day 9. The survival rate decreased to 50% on Day 12, and was maintained at this level throughout the observation period (FIG. 4). The hematocrit values of the infected mice treated with 1,000 U/kg of r-hu-EPO for 8 days increased to 56% on Day 6, and rapidly decreased to 42%, a value lower than the non-treatment control values, on Day 9 (FIG. 5). Trypanosoma infected mice without r-hu-EPO treatment died around the first peak of parasitemia (2.4-10.1 × 10⁴ cells/ml blood). Parasitemia in surviving animals had several distinct peaks as observed in Experiment 1 (FIG. 6). The growth curve of animals was not different among the experimental groups (FIG. 7).

### [Experimental Example 2] Effect by treatment with r-hu-EPO or pentamidine

C57BL/6J female mice (CLEA, Tokyo, Japan) at 8 weeks of age (4 mice/group) were infected with 1,000 cells of T. congolense (International Livestock Research Institute, Kenya) by intraperitoneal injection (Day 0). The mice were treated daily with recombinant human erythropoietin (Chugai Pharmaceuticals, Tokyo, Japan) at a dose of 5,000 U/kg for 8days from Day 0 to Day 7 of infection, or were similarly treated with the existing antiprotozoal agent pentamidine (Benambax: Chugai Pharmaceuticals: hereinafter referred to simply as Pen) at a dose of 4 mg/kg. Their survival rate and hematocrit values were monitored at intervals of 2 days from Day 0.

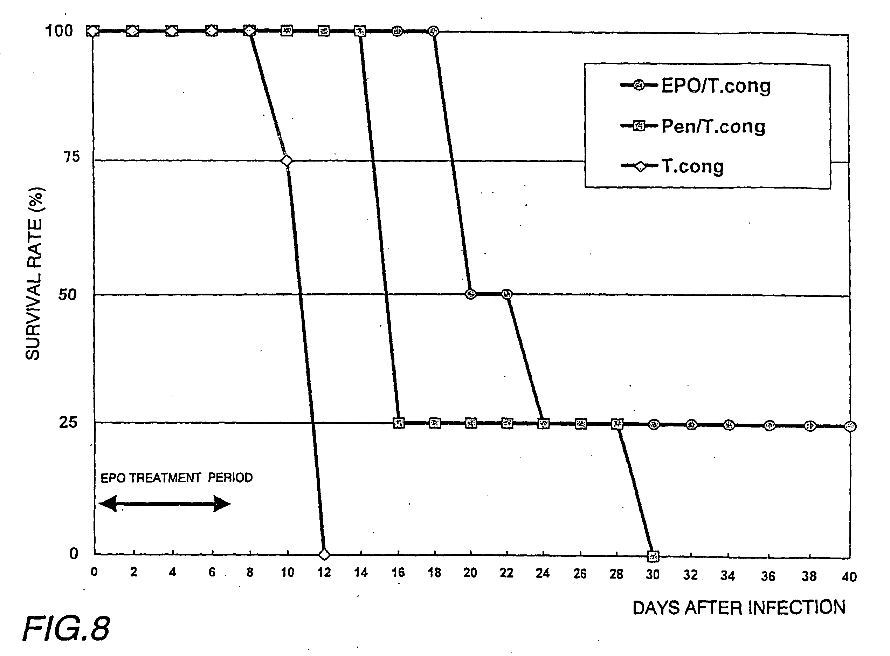

FIG. 8 shows the survival rate of each of the groups, i.e., the infected mice, the infected mice treated with r-hu-EPO, and the infected mice treated with pentamidine (Pen).

The number of days of survival after infection with T. cong. was significantly improved by treatment with EPO or Pen. The effect of EPO was comparable to or higher than that of Pen. However, there was no statistically significant difference between EPO and Pen.

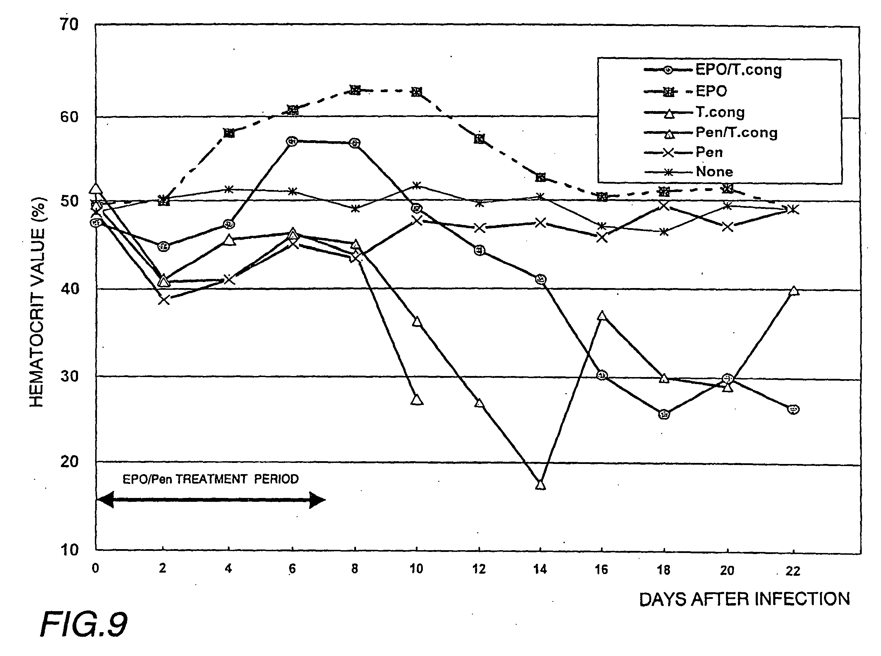

The hematocrit values of the infected mice, the infected mice treated with r-hu-EPO, and the infected mice treated with pentamidine are shown in FIG. 9.

The 8-day treatment with EPO was observed to ameliorate anemia due to T. cong., but this effect was not observed with Pen. Following treatment with Pen, the hematocrit values decreased transiently to about 40%.

In the light of these results, Pen is presumed to exhibit an antiprotozoal effect by the mechanism of action different from that of EPO, because no hematopoietic effect is observed in Pen. Thus, the combined use of EPO and Pen with different mechanisms of action is expected to show a further life-prolonging effect.

Examples concerned with pharmaceutical manufacturing will be shown below.

### [Example 1]

| | |
|---|---|
| Erythropoietin | 8 µg |
| Total amount made with distilled water for injection | 2 ml |

A solution was aseptically prepared from the above formulation. The resulting solution was dispensed in a vial, and sealed up.

### [Example 2]

| | |
|---|---|
| Erythropoietin | 8 µg |
| Total amount made with distilled water for injection | 2 ml |

A solution was aseptically prepared from the above formulation. The resulting solution was dispensed in a vial, lyophilized, and sealed up.

### [Example 3]

| | |
|---|---|
| Erythropoietin | 16 µg |
| Total amount made with distilled water for injection | 2 ml |

A solution was aseptically prepared from the above formulation. The resulting solution was dispensed in a vial, and sealed up.

### [Example 4]

| | |
|---|---|
| Erythropoietin | 16 µg |
| Total amount made with distilled water for injection | 2 ml |

A solution was aseptically prepared from the above formulation. The resulting solution was dispensed in a vial, lyophilized, and sealed up.

### [Example 5]

| | |
|---|---|
| Erythropoietin | 8 µg |
| Human serum albumin | 5 mg |
| Total amount made with distilled water for injection | 2 ml |

A solution was aseptically prepared from the above formulation. The resulting solution was dispensed in a vial, and sealed up.

### [Example 6]

| | |
|---|---|
| Erythropoietin | 8 µg |
| Human serum albumin | 5 mg |
| Total amount made with distilled water for injection | 2 ml |

A solution was aseptically prepared from the above formulation. The resulting solution was dispensed in a vial, lyophilized, and sealed up.

### [Example 7]

| | |
|---|---|
| Erythropoietin | 16 µg |
| Human serum albumin | 5 mg |
| Total amount made with distilled water for injection | 2 ml |

A solution was aseptically prepared from the above formulation. The resulting solution was dispensed in a vial, and sealed up.

### [Example 8]

| | |
|---|---|
| Erythropoietin | 16 µg |
| Gelatin | 5 mg |
| Total amount made with distilled water for injection | 2 ml |

A solution was aseptically prepared from the above formulation. The resulting solution was dispensed in a vial, lyophilized, and sealed up.

### [Examples 9 to 12]

Injections were prepared in the same manner as in Examples 5 to 8, except that 5 mg of dextran 40 was used instead of human serum albumin used in Examples 5 to 8.

### [Example 13]

D-Mannitol (5 g), 1 mg of erythropoietin, and 100 mg of human serum albumin were aseptically dissolved in 100 ml of distilled water for injection to prepare an aqueous solution. The aqueous solution (1 ml) was dispensed in a vial, lyophilized, and sealed up.

### [Example 14]

A solution (1 ml) containing the following ingredients was prepared:

| | |
|---|---|
| EPO | 1,500 IU |
| Nonionic surfactant | 0.05 mg |
| (Polysorbate 80: Nikko Chemicals) | |
| Sodium chloride | 8.5 mg |
| L-Arginine hydrochloride (Sigma) | 10 mg |

This solution was adjusted to pH 6.0 with a 10 mM phosphate buffer solution (Wako Pure Chemical Industries), and 1 ml of the solution was charged into a 5 ml glass vial. The glass vial was stoppered, and sealed to produce a solution preparation.

### [Example 15]

A solution preparation was produced in the same manner as in Example 14 from a solution containing the following ingredients in 1 ml:

| | |
|---|---|
| EPO | 1,500 IU |
| Nonionic surfactant | 0.05 mg |
| (Polysorbate 80: Nikko Chemicals) | |
| Sodium chloride | 8.5 mg |
| L-Histidine hydrochloride (Sigma) | 10 mg |

### [Example 16]

A solution preparation was produced in the same manner as in Example 14 from a solution containing the following ingredients in 1 ml:

| | |
|---|---|
| EPO | 1,500 IU |
| Nonionic surfactant | 0.05 mg |
| (Polysorbate 80: Nikko Chemicals) | |
| Sodium chloride | 8.5 mg |
| L-Lysine hydrochloride (Sigma) | 10 mg |

### [Industrial Applicability]

Pharmaceuticals containing EPO as the active ingredient are effective for the treatment of protozoal diseases, especially protozoal diseases involving anemia and having high lethality rates (for example, trypanosomiasis and malaria). Moreover, these pharmaceuticals show low side effects, and are safe. Furthermore, EPO and protozoa are simultaneously administered to establish protozoal infection without leading to death, whereby their combined use can be utilized as a vaccine.

## Claims

1. An agent for treatment of protozoal disease, containing erythropoietin as an active ingredient.

2. The agent for treatment of protozoal disease according to claim 1, wherein said protozoal disease is a disease accompanied by anemia.

3. The agent for treatment of protozoal disease according to claim 1, wherein said protozoal disease is trypanosomiasis or malaria.

4. The agent for treatment of protozoal disease according to claim 2, wherein said protozoal disease is trypanosomiasis.

5. The agent for treatment of protozoal disease according to claim 1, which is formulated as an injection, with the erythropoietin as the active ingredient.

6. A method or treatment of protozoal disease, comprising administering an effective amount of erythropoietin to a patient with the protozoal disease.

7. The method for treatment according to claim 6, wherein said protozoal disease is a disease accompanied by anemia.

8. The method for treatment according to claim 6, wherein said protozoal disease is trypanosomiasis or malaria.

9. The method for treatment according to claim 8, wherein said protozoal disease is trypanosomiasis.

10. Use of erythropoietin for producing an agent for treatment of protozoal disease.

11. The use of erythropoietin according to claim 10, wherein said agent for treatment of protozoal disease is an injection.
